(19) Europäisches Patentamt
European Patent Office
Office européen des brevets

(11) Publication number: 0 104 598
A1

# (12) EUROPEAN PATENT APPLICATION

(21) Application number: 83109367.9

(22) Date of filing: 21.09.83

(51) Int. Cl.[3]: C 07 C 41/16
C 07 C 43/205, C 07 C 43/225

(30) Priority: 23.09.82 US 422242

(43) Date of publication of application:
04.04.84 Bulletin 84/14

(84) Designated Contracting States:
CH DE FR GB LI

(71) Applicant: PPG INDUSTRIES, INC.
One PPG Place
Pittsburgh Pennsylvania 15272(US)

(72) Inventor: Thompson, Ralph Brewster
Adams Road
Oakbrook Illinois 60521(US)

(74) Representative: Hann, Michael, Dr. et al,
Patentanwälte Dr. Michael Hann Dr. H.-G. Sternagel
Marburger Strasse 38
D-6300 Giessen(DE)

## (54) Method for producing ethers from highly hindered phenols or salts thereof and carbonates.

(57) Ethers are prepared by reacting highly hindered phenols or salts thereof with organic carbonates.

EP 0 104 598 A1

Croydon Printing Company Ltd.

METHOD FOR PRODUCING ETHERS FROM HIGHLY HINDERED PHENOLS OR SALTS THEREOF AND CARBONATES

The alkylation of many phenols is customarily accomplished by reaction of the phenols with alkyl esters of inorganic acids or with chlorocarbonic acid esters. The esters of sulfuric acid and of the hydrogen halides have attained particular importance for this purpose. Dimethyl sulfate and methyl iodide are typical alkylating agents. United States Patent No. 4,192,949 discloses that various phenols may be alkylated or aralkylated using organic carbonates of an aliphatic or araliphatic alcohol. The disclosure of United States Patent No. 4,192,949 is, in its entirety, incorporated herein by reference.

It has now been discovered that when the hydroxyl group of a phenol is highly sterically hindered, that is to say, the phenol is a highly hindered phenol, alkylation through use of dialkyl sulfate or alkyl halide often either produces low yields of the alkylated product or does not produce the product at all. It has been further discovered that many highly hindered phenols, for which conversion to ethers by esters of strong inorganic acids is largely ineffectual or inefficient, may be successfully and efficiently converted to ethers by the use of organic carbonates of at least one primary alcohol. The present invention is therefore

0104598

based on the discovery that the general procedure of United States Patent No. 4,192,949 is particularly applicable to a specific class of phenols or salts thereof, and is accordingly an improvement upon the patent.

More particularly, the present invention is a method comprising reacting highly hindered phenol or salt thereof represented by the formula

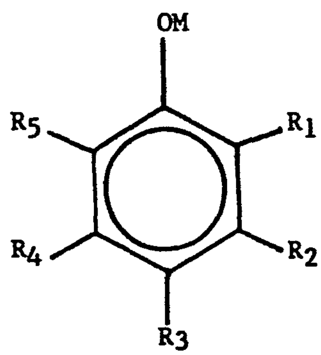

(I)

0104598

wherein

a. $R_1$ is bromo or a group represented by the formula

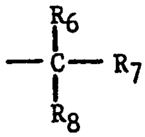

(II)

where $R_6$, $R_7$ and $R_8$ are each independently a monovalent organo group,

b. $R_2$, $R_3$, $R_4$ and $R_5$ are each independently hydrogen or a substituent, and

c. M is hydrogen, a monovalent metal or a monovalent fractional part of a polyvalent metal,

with organic carbonate of at least one primary alcohol to produce ether.

Any of $R_2$, $R_3$, $R_4$ and $R_5$ which are substituents may be any substituents which do not preclude ether formation. They may be the same or they may be different. They may themselves be unreactive under the conditions of the reaction as is the usual case, or one or more of them may be reactive under such conditions. Examples of substituents which may be used include halo, nitro, alkyl, alkoxy, alkoxyalkyl, aryloxy, aralkyl, carboxy, acyloxy, aroyloxy, hydroxy, cyano, cycloalkyl, (cycloalkyl)alkyl, acyl, aroyl, mercapto, alkylthio, seleno, alkylseleno, alkenyl and alkadienyl. The substituents themselves may be substituted, as for example, they may be haloalkyl, halocycloalkyl, nitrobenzyl and the like. Chloro and bromo are typically employed for halo. The alkyl may be straight or branched and usually has from 1 to about 20 carbon atoms. The alkoxy ordinarily has from 1 to about 4 carbon atoms. The alkoxyalkyl generally contains from 1 to 4 carbon atoms in the alkoxy portion and from 1 to about 10 carbon atoms in the alkyl portion. The aryl portion of the aryloxy often contains from 6 to about 10 carbon atoms. The aralkyl often contains from 6 to about 10 carbon atoms in the aryl portion and from 1 to about 10 carbon atoms in the alkyl portion. The acyloxy ordinarily contains from 2 to about 12 carbon atoms. The acyloxy typically contains from 2 to about 6 carbon atoms. The cycloalkyl usually has from about 6 to about 8 carbon atoms. The (cycloalkyl)alkyl generally

contains from about 6 to about 8 carbon atoms in the cycloalkyl portion and from 1 to about 10 carbon atoms in the alkyl portion. The aryl often has from 7 to about 11 carbon atoms. The alkylthio and alkylseleno generally have from 1 to about 4 carbon atoms. The alkenyl typically has from 2 to about 10 carbon atoms. The alkadienyl usually has from about 4 to about 10 carbon atoms.

Frequently many, and sometimes all of $R_2$, $R_3$, $R_4$ and $R_5$ which are substituents are each independently chloro, bromo, unsubstituted alkyl or substituted alkyl. The unsubstituted alkyl and substituted alkyl may be straight chain or they may be branched and they most often contain from one to about ten carbon atoms. From one to about four carbon atoms is preferred.

$R_6$, $R_7$ and $R_8$ may be substantially any monovalent organo groups which do not preclude ether formation. They may be the same, they may be different or two of them may be the same and different from the third. Examples of organo groups which may be used include alkyl, alkenyl, aryl, (cycloalkyl)alkyl, aralkyl and cycloalkyl. Such groups may be substituted or unsubstituted. They may themselves be inert to the conditions of the reaction or they may contain groups such as mercapto, hydroxyl, amino, selenyl or carboxyl, which are reactive with organic carbonate. When an aryl group or a group containing an aryl portion is used, it may be homocyclic or heterocyclic; it may comprise a single ring or it may comprise a ring assembly. The alkyl may be straight or branched and usually contains from 1 to about 20 carbon atoms. The alkenyl typically contains from 2 to about 10 carbon atoms. The aryl often contains from 6 to about 10 carbon atoms; it may be homocyclic or heterocyclic; it may comprise a single ring or a ring assembly. The (cycloalkyl)alkyl generally contains from about 6 to about 8 carbon atoms in the cycloalkyl portion and from 1 to about 10 carbon atoms in the alkyl portion. The aralkyl often contains from 6 to about 10 carbon atoms in the aryl portion and from 1 to about 10 carbon atoms in the alkyl portion. The cycloalkyl usually contains from about 6 to about 8 carbon atoms.

Often, $R_6$, $R_7$ and $R_8$ are each independently an alkyl group. Any alkyl group may be straight or branched, but usually they are all straight. Typically each alkyl group has from 1 to about 20 carbon atoms. Often each alkyl group has from 1 to about 10 carbon atoms. Preferably $R_7$ is an alkyl group having from 1 to about 6 carbon atoms while $R_6$ and $R_8$ are each independently methyl or ethyl. It is especially preferred that $R_7$ have from 1 to about 6 carbon atoms while $R_6$ and $R_8$ are each methyl. The alkyl groups are usually unsubstituted, but any of them may contain one or more substituents of a minor nature that do not render the highly hindered phenol or salt thereof unsuited for its intended purpose. The substituents may be reactive or unreactive under the conditions of the reaction, but usually they are unreactive.

The identity of M may vary widely. Typically M is hydrogen, alkali metal or the monovalent fractional part of alkaline earth metal. Hydrogen, sodium or potassium is most frequently used.

A subclass of highly hindered phenol of particular interest is that of Formula I wherein $R_5$ is a substituent, since this subclass is more highly hindered than when $R_5$ is hydrogen. It is preferred that $R_5$ be chloro, bromo or unsubstituted alkyl.

An especially preferred subclass of highly hindered phenol is that of Formula I wherein $R_5$ is bromo or a group represented by the formula

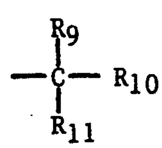

(III)

where the above discussion in respect of $R_6$, $R_7$ and $R_8$ is applicable to $R_9$, $R_{10}$ and $R_{11}$, respectively. The phenol of this subclass is especially highly hindered. In some instances, $R_1$ and $R_5$ are each bromo. In some other instances, $R_1$ is the group of Formula II and $R_5$ is the group of Formula III.

The organic carbonate of at least one primary alcohol used in the method is subject to wide variation. A class of particular importance may be represented by the formula

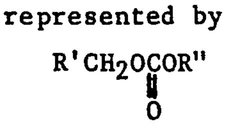

(IV)

wherein R' is hydrogen, alkyl, alkenyl, aralkyl, (cycloalkyl)alkyl, cycloalkyl or lower aryl, and R" is alkyl, alpha,beta-saturated alkenyl, aralkyl, (cycloalkyl)alkyl, cycloalkyl or lower aryl. When alkyl is employed, it usually has from 1 to about 20 carbon atoms, often from 1 to about 10 carbon atoms. Lower alkyl having from 1 to about 4 carbon atoms is preferred. Methyl and ethyl are especially preferred. When aralkyl is employed, the aryl portion generally contains from 6 to about 10 carbon atoms and the alkyl portion usually contains from 1 to about 10 carbon atoms; benzyl is preferred. When (cycloalkyl)alkyl is used, the cycloalkyl portion generally contains from about 6 to about 8 carbon atoms and the alkyl portion typically contains from 1 to about 10 carbon atoms; 10 is preferred. The cycloalkyl typically has from about 6 to about 8 carbon atoms; cyclohexyl is preferred. The lower aryl usually has from 6 to about 10 carbon atoms; phenyl is preferred. When alkenyl is used for R', it usually contains from 2 to about 9 carbon atoms; vinyl is preferred. When alpha,beta-saturated alkenyl is employed for R", it usually contains from 3 to about 10 carbon atoms; allyl is preferred. These groups are usually unsubstituted, although one or more minor substituents which do not render the organic carbonate unsuitable for its intended purpose may be present on any of the groups. Similarly, those groups having one or more rings are usually homocyclic, but one or more hetero atoms may be present so long as they do not preclude ether formation. The aliphatic groups and the aliphatic portions of hybrid groups such as aralkyl may be straight or branched, but it is preferred they be straight. Only one organic carbonate or a plurality of organic carbonates may be used as desired.

Examples of organic carbonates which may be employed include dimethyl carbonate, ethyl methyl carbonate, diethyl carbonate, propyl methyl carbonate, isopropyl methyl carbonate, isopropyl ethyl carbonate, butyl methyl carbonate, secondary-butyl methyl carbonate, isobutyl methyl carbonate, tertiary-butyl methyl carbonate, cyclohexyl methyl carbonate, benzyl methyl carbonate, phenyl methyl carbonate and diallyl carbonate. It is preferred that R' be

hydrogen or methyl. The particularly preferred organic carbonates are dimethyl carbonate and diethyl carbonate.

The reaction of highly hindered phenol or salt thereof and organic carbonate is usually conducted in the liquid phase. It may be carried out batchwise, continuously, semibatchwise or semicontinuously. When the organic carbonate is a liquid under the conditions of the reaction, it often acts as a solvent for the highly hindered phenol or salt thereof. Typically, but not necessarily, excess organic carbonate is employed and this usually serves to dissolve the highly hindered phenol or salt thereof throughout the reaction. In many cases, one or more by-products of the reaction, most notably alcohols, also tend to dissolve the highly hindered phenol or salt thereof. Although extrinsic solvent is not ordinarily employed, it may be used when desired or when necessary to dissolve one or more of the reactants. Examples of suitable extrinsic solvents include methanol, ethanol, acetonitrile, benzene, toluene, dioxane, dimethylformamide and chlorinated solvents such as chloroform, methylene chloride, ethylene chloride, carbon tetrachloride and chlorobenzene. Only one extrinsic solvent or a plurality of extrinsic solvents may be used as desired. For many reactions, extrinsic solvent need not be introduced, and the reaction may be neat.

When extrinsic solvent is used, the weight ratio of extrinsic solvent to the highly hindered phenol or salt thereof initially present is subject to wide variation. Generally, the amount of solvent should be sufficient to dissolve the reactants at the reaction temperature. The weight ratio of extrinsic solvent, when used, to the highly hindered phenol or salt thereof initially present is usually in the range of from about 0.01:1 to about 20:1. From about 0.1 to about 5:1 is preferred.

The temperatures at which the reaction is conducted may vary widely, but ordinarily they are in the range of from about 90°C to about 240°C. Preferably the temperatures are in the range of from about 160°C to about 210°C.

The pressures at which the reaction is conducted are similarly susceptible to wide variation. Atmospheric and superatmospheric pressures are generally employed, although lesser pressures may sometimes be used. Generally the pressure is in the range of from about zero to about 5000 kilopascals, gauge, but higher pressures may be used. Preferably the pressure is in the range of from about 700 to about 3500 kilopascals, gauge.

The reaction is generally conducted in the presence of catalyst. Exemplary catalysts which may be used include nitrogen-containing heterocyclic catalysts such as pyridine, 4-(dimethylamino)pyridine, imidazole, 2,6-lutidine and 2,4,6-collidine. Other suitable catalysts include the tertiary amines, tertiary amine salts, diamines, diamine salts, quaternary ammonium salts, tertiary phosphines and others disclosed in United States Patent No. 4,192,949. Only a single catalyst or a mixture of catalysts may be used where desired. The preferred catalyst is 4-(dimethylamino)-pyridine. In some cases, catalyst need not be used.

The equivalent ratio of the catalyst to the highly hindered phenol or salt thereof initially present may vary widely but usually it is in the range of from about 0.005:1 to about 0.5:1. It is preferred that the equivalent ratio be in the range of from about 0.01:1 to about 0.2:1.

A preferred embodiment of the invention is the method for alkylating highly hindered phenol or salt thereof represented by Formula I or Formula II, discussed above, comprising reacting the phenol or salt thereof with dialkyl carbonate in which at least one of the alkyl groups of the dialkyl carbonate is unsubstituted methyl or is an alkyl group having at least two carbon atoms wherein the number one carbon atom of the alkyl group is attached to two hydrogen atoms, to produce ether.

The alkyl groups of the dialkyl carbonate used in this embodiment may be the same or different; it is preferred that they be the same. Typically each alkyl group independently contains from 1 to about 20 carbon atoms, often from 1 to about 10 carbon atoms. Lower alkyl having from 1 to about 4 carbon atoms is

preferred. Methyl and ethyl are especially preferred. The alkyl groups of the dialkyl carbonate are usually unsubstituted, although either or both may, subject to the conditions of the immediately preceding paragraph, contain minor substituents which do not render the compound unsuitable for its intended purpose. Only one or a mixture of dialkyl carbonates may be used as desired. The preferred dialkyl carbonates are dimethyl carbonate and diethyl carbonate; dimethyl carbonate is particularly preferred.

Following preparation, the ether may be recovered from the reaction mixture by any of the various techniques known to the art. Distillation at reduced pressure is one such technique that is frequently employed.

The present invention may be employed to produce ethers having widely varying uses, as for example, as intermediates for the manufacture of pharmaceuticals, dyes, crop protection agents and scents.

Although it is ordinarily permissible to practice the invention in a rather strongly acidic or strongly basic medium, it is preferred to conduct the method at, or somewhat near, neutrality. The avoidance of strongly acidic or strongly basic media is an especial advantage when the highly hindered phenol or salt thereof contains one or more functional groups which react adversely under such extreme conditions.

The invention is further described in conjunction with the following examples which are to be considered illustrative rather than limiting.

EXAMPLE I

A 1-liter reactor equipped with an agitator, an automatic temperature regulator, a pressure gauge and an electric heating mantle was charged with 55 grams of 2,6-di-tert-butyl-4-methylphenol, 150 milliliters of dimethyl carbonate and 4 grams of 4-(dimethylamino)pyridine. The reactor was sealed and heated while the contents were agitated. The temperatures at various times after heating was begun are shown in Table 1. Due to malfunction of the pressure gauge, reliable pressure readings were not obtained.

Table 1

| Time, Hours:Minutes | Temperature, °C | Remarks |
|---|---|---|
| 0:00 | Ambient | Heat On |
| 1:13 | 180 | |
| 2:33 | 180 | |
| 3:58 | 180 | Heat Off |

The reactor was allowed to cool to ambient temperature at which time the gas within the reactor was under a positive pressure. Analysis of a sample of the gas showed that carbon dioxide was present. The pressure was released and 200 grams of liquid was recovered from the reactor. A small sample was heated on a water both to evaporate a portion of the liquid. Upon cooling the liquid residue to room temperature, no solid formation was observed. Gas-liquid chromatographic analysis of the liquid from the reactor showed it to contain 64.7 area percent dimethyl carbonate, 24.5 area percent 1-methoxy-2,6-di-tert-butyl-4-methylbenzene, 7.5 area percent methanol and 2.2 area percent 2,6-di-tert-butyl-4-methylphenol.

The remaining liquid from the reactor was placed in a distillation apparatus and most of the dimethyl carbonate and methanol were removed overhead. A vacuum was then applied to the stripped liquid and the fraction distilling in the range of 80 to 81°C at an absolute pressure of about 67 pascals was collected as liquid product. The collected product weighed 51.5 grams. Gas-liquid chromatographic analysis showed the product to contain 94.54 area percent 1-methoxy-2,6-di-tert-butyl-4-methylbenzene and 5.24 area percent 2,6-di-tert-butyl-4-methylphenol.

EXAMPLE II

A 1-liter reactor equipped with an agitator, an automatic temperature regulator, a pressure gauge and an electric heating mantle was charged with 55 grams of 2,6-di-tert-butyl-4-methylphenol and 150 milliliters (160 grams) of dimethyl carbonate and 3 grams of 4-(dimethylamino)pyridine. The reactor was sealed and heated while the contents were agitated. The temperatures and pressures at various times after heating was begun are shown in Table 2.

Table 2

| Time, Hours:Minutes | Temperature, °C | Pressure Kilopascals, Gauge | Remarks |
|---|---|---|---|
| 0:00 | Ambient | 0 | Heat On |
| 0:20 | 115 | 414 | |
| 0:35 | 200 | 1862 | |
| 0:55 | 200 | 2689 | |
| 2:15 | 200 | 3378 | |
| 3:15 | 200 | 3585 | Heat Off |

The reactor was allowed to cool to ambient temperature at which time the pressure was 1300 kilopascals gauge. Analysis of a sample of the gas showed that carbon dioxide was present. The pressure was released and 183.3 grams of black liquid was recovered from the reactor. Gas-liquid chromatographic analysis showed the liquid to contain 59.6 area percent dimethyl carbonate, 28.4 area percent 1-methoxy-2,6-di-tert-butyl-4-methylbenzene, 10.1 area percent methanol and 0.07 area percent 2,6-di-tert-butyl-4-methylphenol.

The liquid from the reactor was placed in a distillation apparatus and most of the dimethyl carbonate and methanol were removed overhead. A vacuum was applied to the stripped liquid and the fraction distilling in the range of 84 to 85°C at an absolute pressure of 133 pascals was collected as product. The collected product weighed 52.1 grams. Gas-liquid chromatographic analysis showed the product to contain 99.56 area percent 1-methoxy-2,6-di-tert-butyl-4-methylbenzene.

## EXAMPLE III

The reactor of Example II was charged with 67.5 grams of pentabromophenol, 160 grams of dimethyl carbonate and 3 grams of 4-(dimethylamino)pyridine. The reactor was sealed and heated while the contents were agitated. The temperatures and pressures at various times after heating was begun are shown in Table 3.

Table 3

| Time, Hours:Minutes | Temperature, °C | Pressure, Kilopascals, Gauge | Remarks |
|---|---|---|---|
| 0:00 | Ambient | 0 | Heat On |
| 0:20 | 125 | 276 | |
| 0:55 | 180 | 2482 | |
| 1:50 | 180 | 2965 | |
| 2:35 | 180 | 3172 | |
| 4:00 | 180 | 3241 | Heat Off |

The reactor was allowed to cool to ambient temperature. Analysis of a sample of the gas within the reactor showed that carbon dioxide was present. The pressure was released and the reaction mixture was observed to contain both liquid and solids. The solid was not soluble in warm dilute aqueous sodium hydroxide. Most of the reaction mixture was removed from the reactor, however not all of the solids were recovered. The removed reaction mixture was evaporated on a steam both to yield 64.5 grams of crude solid.

The crude product was quite soluble in xylene and the color was improved by charcoal. It was slightly soluble in hot ethanol and crystallized poorly from a mixture of toluene and normal heptane. Infrared and nuclear magnetic resonance analyses showed the crude product to be chiefly methoxypentabromobenzene. Although the present invention has been described with reference to specific details of certain embodiments thereof, it is not intended that such details should be regarded as limitations upon the scope of the invention except insofar as they are included in the accompanying claims.

CLAIMS:

1. A method comprising reacting highly hindered phenol or salt thereof represented by the formula

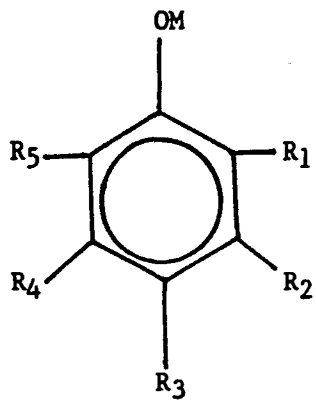

(I)

wherein

a. $R_1$ is bromo or a group represented by the formula

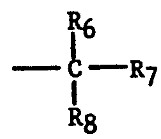

where $R_6$, $R_7$ and $R_8$ are each independently a monovalent organo group,

b. $R_2$, $R_3$, $R_4$ and $R_5$ are each independently hydrogen or a substituent, and

c. M is hydrogen, a monovalent metal or a monovalent fractional part of a polyvalent metal,

with organic carbonate of at least one primary alcohol to produce ether.

2. The method of Claim 1 wherein

a. $R_2$, $R_3$, $R_4$ and $R_5$ are each independently hydrogen, halo, nitro, alkyl, alkoxy, alkoxyalkyl, aryloxy, aralkyl, carboxy, acyloxy, aroyloxy, hydroxy, cyano, cycloalkyl, (cycloalkyl)alkyl, acyl, aroyl, mercapto, alkylthio, seleno, alkylseleno, alkenyl or alkadienyl, and

b. $R_6$, $R_7$ and $R_8$ are each independently alkyl, alkenyl, aryl, (cycloalkyl)alkyl, aralkyl or cycloalkyl.

3. The method of Claim 2 wherein $R_2$, $R_3$, $R_4$ and $R_5$ are each independently hydrogen, chloro, bromo, unsubstituted alkyl or substituted alkyl.

4. The method of Claim 1 wherein $R_1$ is bromo.

5. The method of Claim 4 wherein $R_5$ is bromo.

6. The method of Claim 1 wherein $R_1$ is a group represented by the formula

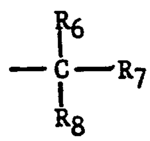

where $R_6$, $R_7$ and $R_8$ are each independently a monovalent organo group.

7. The method of Claim 6 wherein $R_6$, $R_7$ and $R_8$ are each independently alkyl, alkenyl, aryl, (cycloalkyl)alkyl, aralkyl or cycloalkyl.

8. The method of Claim 6 wherein $R_6$, $R_7$ and $R_8$ are each independently alkyl.

9. The method of Claim 6 wherein $R_6$, $R_7$ and $R_8$ are each independently straight alkyl.

10. The method of Claim 6 wherein $R_7$ is an alkyl group having from 1 to about 6 carbon atoms and $R_6$ and $R_8$ are each independently methyl or ethyl.

11. The method of Claim 6 wherein $R_5$ is a group represented by the formula

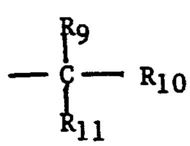

wherein $R_9$, $R_{10}$ and $R_{11}$ are each independently a monovalent organo group.

12. The method of Claim 11 wherein $R_9$, $R_{10}$ and $R_{11}$ are each independently alkyl, alkenyl, aryl, (cycloalkyl)alkyl, aralkyl or cycloalkyl.

13. The method of Claim 11 wherein $R_6$, $R_7$, $R_8$, $R_9$, $R_{10}$ and $R_{11}$ are each independently alkyl.

14. The method of Claim 11 wherein $R_6$, $R_7$, $R_8$, $R_9$, $R_{10}$ and $R_{11}$ are each independently straight alkyl.

15. The method of Claim 11 wherein $R_7$ and $R_{10}$ are each independently an alkyl group having from 1 to about 6 carbon atoms and $R_6$, $R_8$, $R_9$ and $R_{11}$ are each independently methyl or ethyl.

16. The method of Claim 1 wherein $R_5$ is a group represented by the formula

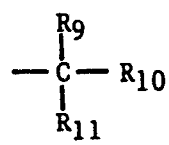

wherein $R_9$, $R_{10}$ and $R_{11}$ are each independently a monovalent organo group.

17. The method of Claim 1 wherein M is hydrogen or alkali metal.

18. The method of Claim 1 wherein M is hydrogen, sodium or potassium.

19. The method of Claim 1 wherein said organic carbonate is represented by the formula

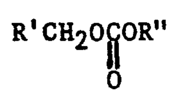

wherein

a. R' is hydrogen, alkyl, alkenyl, aralkyl, (cycloalkyl)alkyl, cycloalkyl or lower aryl, and

b. R" is alkyl, alpha,beta-saturated alkenyl, aralkyl, (cycloalkyl)alkyl, cycloalkyl or lower aryl.

20. The method of Claim 19 wherein

a. R' is hydrogen, alkyl having from 1 to about 20 carbon atoms, alkenyl containing from 2 to about 9 carbon atoms, aralkyl wherein the aryl portion contains from 6 to about 10 carbon atoms and the alkyl portion contains from 1 to about 4 carbon atoms, (cycloalkyl)alkyl wherein the cycloalkyl portion contains from about 6 to about 8 carbon atoms and the alkyl portion contains from 1 to about 4 carbon atoms, cycloalkyl having from about 6 to about 8 carbon atoms or lower aryl having from 6 to about 10 carbon atoms; and

b. R" is alkyl having from 1 to about 20 carbon atoms, alpha,beta-saturated alkenyl containing from 3 to about 10 carbon atoms, aralkyl wherein the aryl portion contains from 6 to about 10 carbon atoms and the alkyl portion contains from 1 to about 4 carbon atoms, (cycloalkyl)alkyl wherein the cycloalkyl portion contains from about 6 to about 8 carbon atoms and the alkyl portion contains from 1 to about 4 carbon atoms, cycloalkyl having from about 6 to about 8 carbon atoms or lower aryl having from 6 to about 10 carbon atoms.

21. The method of Claim 1 wherein said reaction is conducted in the liquid phase.

22. The method of Claim 21 wherein the reaction is conducted in the presence of catalyst.

23. The method of Claim 22 wherein said catalyst is nitrogen-containing heterocyclic catalyst.

24. The method of Claim 22 wherein said catalyst is 4-(dimethylamino)pyridine.

25. The method of Claim 22 wherein said catalyst is pyridine.

26. The method of Claim 22 wherein the equivalent ratio of said catalyst to said phenol or salt thereof initially present is in the range of from about 0.005:1 to about 0.5:1.

27. The method of Claim 21 wherein said reaction is conducted at a temperature in the range of from about 90°C to about 240°C.

28. The method of Claim 21 wherein said reaction is conducted at a pressure in the range of from about zero to about 5000 kilopascals, gauge.

29. The method of Claim 21 wherein said reaction is conducted in the presence of extrinsic solvent.

30. The method of Claim 29 wherein the weight ratio of said extrinsic solvent to said phenol or salt thereof initially present is in the range of from about 0.01:1 to about 20:1.

31. The method of Claim 21 wherein said reaction is neat.

0104598

European Patent Office

# EUROPEAN SEARCH REPORT

Application number

EP 83 10 9367

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 000 162 (BASF)<br><br>* Whole document *<br>--- | 1,2,6-10,17-31 | C 07 C 41/16<br>C 07 C 43/205<br>C 07 C 43/225 |
| A | GB-A-2 026 484 (ANIC)<br>* Claims *<br>--- | 1 | |
| A | US-A-3 870 744 (E.R. WAGNER)<br>* Claims *<br>--- | 1 | |
| A | US-A-4 341 905 (P.E. STREGE)<br>* Claims; examples 15-29 *<br>----- | 1 | |
| | | | TECHNICAL FIELDS SEARCHED (Int. Cl. 3) |
| | | | C 07 C 41/00<br>C 07 C 43/00 |

The present search report has been drawn up for all claims

| Place of search | Date of completion of the search | Examiner |
|---|---|---|
| THE HAGUE | 18-11-1983 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document